# EUROPEAN PATENT APPLICATION

(11) **EP 2 628 538 A1**
(43) Date of publication of application: **21.08.2013**
(21) Application number: 12305183.1
(22) Date of filing: 17.02.2012
(51) Int. Cl.: B01J 23/80, B01J 37/03, C07C 29/154

(54) **Process for manufacturing a methanol synthesis catalyst**

(71) Applicant: TOTAL RAFFINAGE CHIMIE, 92400 Courbevoie (FR)
(72) Inventor: Robles Macias, Luis Angel, 75015 Paris (FR); Curulla-Ferre, Daniel, 92400 Courbevoie (FR); Ferreira, Cristina, 75015 Paris (FR); Santiago Redondo, Marta, 8038 Zürich (CH); Stoica, Georgiana, 43006 Tarragona (ES)
(74) Representative: Cabinet Plasseraud

(57) **Abstract**

The present invention pertains to a process for the manufacture of a CuO/ZnO/Al₂O₃ catalyst, which may be used in the synthesis of methanol from syngas. It also pertains to the catalyst thus obtained, which offers a high external surface area, fine copper oxide crystallites and a high copper surface after reduction.

## Description

### FIELD OF THE INVENTION

The present invention pertains to a process for the manufacture of a CuO/ZnO/Al₂O₃ catalyst, which may be used in the synthesis of methanol from syngas. It also pertains to the catalyst thus obtained, which offers a high external surface area, fine copper oxide crystallites and a high copper surface after reduction.

### BACKGROUND OF THE INVENTION

Methanol is widely used in different applications such as: the synthesis of formaldehyde, which is then involved in the manufacture of plastic materials, paints or textiles, for instance; the production of dimethylether, which may be used in aerosols or as an alternative fuel for diesel engines; the transesterification of triglycerides to produce biodiesel; or as a solvent or a fuel for engines.

Industrially, methanol is generally made catalytically from syngas containing carbon monoxide, carbon dioxide and hydrogen, which is itself produced by steam reforming of natural gas or other hydrocarbons. A widely used catalyst is a Cu/ZnO/Al₂O₃ catalyst described, for instance, in GB-1,159,035. In this ternary system, Zn acts as a promoter and prevents the sintering of copper which may occur above 250°C, while Al₂O₃ provides structural integrity, contributes to an increased surface area and along with ZnO, separates the copper crystallites while inhibiting the sintering, thus inducing the formation of smaller particles.

This catalyst may be prepared by co-precipitation. To this end, nitrate salts of the metals are mixed with a carbonate, bicarbonate, hydroxide, or mixtures thereof of an alkali metal used as a precipitant, at a pH of from 6 to 9, preferably from 6.5 to 7.5, such as 7.1, and at a temperature of from 50 to 100°C, for instance of 85°C. The slurry obtained is then filtered, washed and dried to a powder which is calcined to obtain the required catalyst, generally in the form of pellets. Before use in methanol synthesis, the catalyst is activated in-situ by partially reducing it so as to convert copper oxide into metallic copper.

It is known that the metal oxide precursors formed in a precipitation process may comprise mixed metal carbonates, including hydroxycarbonates of Cu and Zn, such as aurichalcite, which corresponds to (Cu,Zn)₅(CO₃)₂(OH)₆, or else (Cu,Zn)₂(OH)₂CO₃, including zincian malachite and rosasite, or hydrotalcite-like compounds, which are hydrated hydroxycarbonates of Cu and/or Zn and Al, and also single metal carbonates and hydroxycarbonates, namely malachite, Cu₂CO₃(OH)₂, and hydrozincite, Zn₅(OH)₆(CO₃)₂. After calcination at 300-500°C, these precursors may lead to the corresponding metal oxides (WO 2010/146380; Applied Catalysis 1988, 36, 1-65).

The reaction is usually conducted in a batch process, as in WO 2010/146380 or GB 1 159 035. This process traditionally suffers from two important drawbacks. One of them is related to the occurrence of pH gradients due to ineffective stirring of the large reactor volume. Most importantly, due to the discontinuous operation, the residence time of the precipitate particles and concentration of reactants change throughout the precipitation process. In addition, the crystal growth varies between the start and end products. This prevents a constant product quality being maintained throughout the whole precipitation process, which is often essential for the subsequent application of the resulting materials. In essence, batch co-precipitation leads to broad particle size distribution and sub-optimal intermetallic dispersion (since pH gradients may cause precipitation of side phases), which can highly affect the quality of the resultant catalyst. Moreover, in the process disclosed in GB 1 159 035, the reaction temperature is well above 40°C, resulting in large crystallites of the metal oxide precursors, which may furthermore lead to agglomerates depending on the residence time of the precipitate. These large crystallites are not favourable to the activity and durability of the catalyst.

Continuous processes have also been contemplated. They provide a high homogeneity of the final product and allow a better control of the process, especially of the reaction time. Examples of continuous processes have been disclosed in KALUZA S. et al. in ChemCatChem 2011, 3, 189-199. In this reference, continuous precipitation of Cu, Zn and Al precursors using micro-mixers was reported. In a first attempt, single-step continuous precipitation of a mixed solution of the three metal nitrates with Na₂CO₃ was performed, using a residence time of about one second, followed by immediate spray-drying, and then either by calcination, washing and drying, or by washing, drying and calcination. This led to the formation of a non-homogeneous phase containing CuO and Zn-Al amorphous hydrotalcite-like species with a very low specific copper surface area of about 1 m²/g or 6 m²/g, respectively. In a second attempt, consecutive precipitation of the ternary system was approached, at a temperature of preferably 65°C and at a pH of 6.5-7, which resulted in a higher copper surface area (around 20 m²/g). In case the precipitation was followed by aging for one hour, a dried precursor comprising ternary hydrotalcite, zincian malachite and aurichalcite was formed, which enabled the formation of a more active catalyst. Calcination of these samples at 320°C led to CuO and residual carbonates in the form of crystalline malachite-like phase, which means that the complete conversion of the hydroxy carbonate-containing precursors into oxides was not achieved. Moreover, the copper surface after reduction was 15.3 m²/g only, two times lower than the commercial catalyst used in methanol synthesis and obtained by batch precipitation. Finally, the sequential precipitation impacts the cost of the process, since it requires more synthetic units and steps.

Therefore, there remains the need for a cost-effective process for preparing a ternary Cu/Zn/Al catalyst suitable for methanol synthesis, which leads to a catalyst having high external surface area and copper surface area and a small CuO crystallite size which are known to result in high catalyst activity and stability. To satisfy this need, the inventors have developed a simple continuous process which allows controlling the parameters of the precipitation so as to favour the formation of malachite-type precursors, including rosasite and malachite, since the latter have proven to be responsible for the above-mentioned properties after calcination and reduction. Such a continuous process has already been described in WO 2007147881 in connection with the synthesis of catalysts such as hydrotalcite having an increased crystallite size, surface area and porosity, from aqueous solutions of magnesium and aluminum nitrates and a precipitant at pH 10, with a residence time of about 12-18 seconds. However, it has not been suggested to alter the conditions of this process in such a way as to prepare malachite-type precursors of Cu/ZnO/Al₂O₃ catalysts.

### SUMMARY OF THE INVENTION

In one aspect, the present invention is thus directed to a continuous process for the manufacture of a CuO/ZnO/Al₂O₃ catalyst, comprising the successive steps of:
(a) continuously co-reacting aqueous solutions of copper, aluminum and zinc salts with at least one base in a reaction vessel, under stirring, at a temperature ranging from 20 to 40°C, so as to form a slurry, wherein the reaction time is controlled and adjusted between 5 and 60 seconds and the pH of the slurry is adjusted between 7.5 and 8.5,
(b) optionally, subjecting said slurry to aging in a separate vessel,
(c) filtering and washing said slurry with water until a filter cake having a pH of 7 is obtained,
   in such a way as to obtain crystalline metal oxide precursors at the end of step (b), if present, or at the end of step (c),
(d) drying said filter cake so as to obtain a precursor powder, and
(e) calcining said precursor powder at a temperature of less than 320°C to obtain the catalyst.

In another aspect, this invention pertains to the catalyst obtainable according to the above-mentioned process.

In yet another aspect, this invention pertains to the use of this catalyst in the synthesis of methanol from syngas or in a water gas shift reaction.

### DETAILED DESCRIPTION

This invention will now be described in further details. In the following description, the expression "comprised between" should be understood to designate the range of values identified, including the lower and upper bounds.

As mentioned above, the continuous process of this invention comprises a first step of continuously co-reacting aqueous solutions of copper, aluminum and zinc salts with at least one base in a reaction vessel. The salts may be chosen from nitrates, carbonates, acetates and oxalates, preferably nitrates. As a base, mention can be made of carbonates such as sodium carbonate, ammonium carbonate and potassium carbonate; mixtures of at least one hydroxide such as sodium hydroxide and ammonium hydroxide with at least one carbonate ; and mixtures of at least one hydroxide such as sodium hydroxide and ammonium hydroxide with at least one bicarbonate. Sodium carbonate is preferred in this invention. According to a preferred embodiment of this invention, the metals are mixed in respective molar amounts of from 30 to 75 mol.% of Cu, from 20 to 60 mol.% of Zn and 5 to 20 mol.% of Al, provided that the total molar amount of Cu, Zn and Al is 100%, preferably in respective molar amounts of Cu/Zn/Al of 60:30:10. The reactants (aqueous solutions of metal salts and base) may be heated or not before introducing them in the reaction vessel. The reaction is thus conducted at a temperature ranging from 20 to 40°C, so as to form a slurry. Higher temperatures would lead to larger particles, which are not desired.

The reaction vessel is typically a micro-reactor comprising a chamber of a few cubic centimeters (such as from 0.5 to 20 cm³ and preferably from 5 to 10 cm³) fed with separate pipes with reactant solutions and provided with means for stirring suitable for creating a turbulent regime in the reaction chamber (such as a high-shear homogeneizer). The reactants are introduced at selected flow rates and the product is extracted at a selected flow rate, so as to control the residence time within the chamber and adjust it to a pre-selected value. According to this invention, the residence time (i.e. the time during which the product formed remains in the chamber, corresponding to the time lapsing between the introduction of the reactants into the chamber and the extraction of the product therefrom) should be adjusted between 5 and 60 seconds, preferably between 10 and 30 seconds and more preferably between 15 and 20 seconds. Moreover, the precipitation should be performed under stirring, preferably at a rate of from 10.000 to 25.000 rpm so as to avoid local heating points in the reaction vessel, which would accelerate both crystallite growth and particle agglomeration. More importantly, the pH of the slurry formed should be adjusted between 7.5 and 8.5 so as to ensure the formation of malachite-type precursors. The pH is usually monitored by an in-line probe located at the outlet of the reaction vessel.

In the second step of the process according to this invention, the slurry obtained in the first step may be subjected to aging in a separate vessel, although this step is not necessary, as evident from the following Examples.

In any case, the slurry is then filtered and washed with water until a filter cake having a pH of 7 is obtained. It has been observed that the amorphous compounds (such as georgite) formed in the first step of the process do not need prolonged aging to crystallize (for instance into rosasite) and that washing the filter cake with water for a few minutes is enough to induce crystal growth without the need for an intermediate ageing step. The aging step, if present, and the washing step are preferably performed during a combined period of time of at least 15 minutes and usually less than 2 hours. The precipitate obtained according to this invention may be characterized in that it contains connected Cu-Zn phases. More specifically, in the crystalline metal oxide precursors obtained, copper is only included in malachite-type compounds of formula CuMCO₃(OH)₂ where M is Cu or Zn. Preferably, the crystalline metal oxide precursors are malachite, rosasite and copper zinc aluminum carbonate hydroxide hydrate. These metal oxide precursors appear more favourable to the activity of the catalyst formed therefrom than other precursors such as the combination of zincian malachite, aurichalcite and ternary hydrotalcite disclosed by KALUZA S. et al. in ChemCatChem 2011, 3, 189-199. This lower activity of hydrotalcite seems to be related to the fact that, after calcination and reduction, hydrotalcite provides small copper particles which are, similarly to aurichalcite, embedded as clusters in the oxidic matrix. In aurichalcite, copper is atomically dispersed in a zinc hydroxycarbonate matrix, whereas in rosasite, part of the copper atoms are substituted by zinc in the typical malachite structure.

The filter cake is then dried by any appropriate means, such as by oven or spray-drying, at a temperature of about 60 to 100°C, for instance 70 to 85°C, so as to obtain a precursor powder, which is calcined at a temperature of less than 320°C to obtain the catalyst. It should be noted that the precursor powder need not be calcined at higher temperature to be converted into oxides. This is advantageous over typical processes, not only from an economical point of view, but also for obtaining copper oxide particles having a low particle size with a good distribution within the catalyst structure, which in turn allows for a higher activity of the catalyst.

This invention also pertains to the catalyst obtainable according to the process described above. This catalyst is characterized by an external surface area of at least 40 m²/g, preferably of at least 50 m²/g and in that the copper oxide crystallites, which are homogenously distributed within the catalyst, have an average particle size of less than 15 nm, preferably less than 10 nm and more preferably less than 7 nm.

This catalyst may also contain minor amounts of promoters such as Li, Na, K, Rb, Cs, Ba, Mg, Cr, Mn, V, Ti, Zr, Ta, Mo, W, Si or rare earth compounds, which may be introduced by adding a soluble source thereof in the mother solution of Cu-Zn-Al salts. Alternatively, the promoter can be added by incipient wetness impregnation after the co-precipitation (prior to calcination).

The powder catalyst of this invention will usually be formed into agglomerated shape units, by any appropriate method such as: pre-compaction and pelleting of the reduced powder to form pellets, combination with one or more binders and tumbling to form granules, or conversion into a slurry which is then kneaded and extruded into an extrudate, for instance.

Moreover, before using it, the catalyst of this invention will be activated according to known procedures, i.e. by reducing copper oxide into metallic copper without affecting the alumina and zinc oxide. This reduction may be performed by contacting the catalyst with a hydrogen-containing gas (such as N₂), at a temperature of from 160 to 250°C, for instance at a temperature of from 160 to 230°C, under a pressure of from 5 to 10 bars. This catalyst generally has, after reduction, a copper surface area exceeding 16 m²/g and preferably of more than 20 m²/g.

The reduced catalyst may then be used in the synthesis of methanol from syngas or in a water gas shift reaction. The reactions defining the equilibrium compositions of gas mixtures used for the synthesis of methanol are the following: 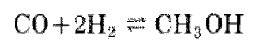 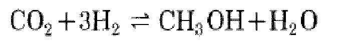 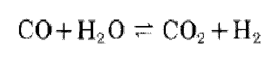 wherein the third one corresponds to the water-gas shift reaction.

The catalyst obtained according to this invention is especially suitable for methanol synthesis. In particular, it has been shown that it has a high activity even when the synthesis gases contain carbon dioxide.

### BRIEF DESCRIPTION OF THE DRAWINGS

This invention will be further understood in light of the following non-limiting examples which are given for illustration purposes only, and also in connection with the attached drawing in which:
- Figure 1 illustrates the XRD patterns of as-synthesized samples derived from continuous and batch syntheses, and
- Figure 2 illustrates the XRD patterns of calcined samples derived from continuous and batch syntheses.

### EXAMPLES

### Example 1: Preparation of a Cu/ZnO/Al₂O₃ catalyst by continuous co-precipitation

Aqueous solutions of copper nitrate (Cu(NO₃)₂·6H₂O), zinc nitrate (Zn(NO₃)₂·5H₂O), and aluminum nitrate (Al(NO₃)₂·9H₂O) and a precipitating agent (Na₂CO₃) were pumped simultaneously into a 6 cm³-reactor attached to a high-shear homogenizer rotating at 13,500 rpm. The pH of the slurry, *i.e*. 8, was measured and controlled by an in-line probe directly at the outlet of the precipitation chamber. The synthesis was carried out with an average residence time of 18 s. Once the precipitation was completed, the slurry was either aged at 60°C for 1 h under mechanical stirring (500 rpm), filtered and thoroughly washed with deionized water until the pH became neutral, or directly filtered and washed.

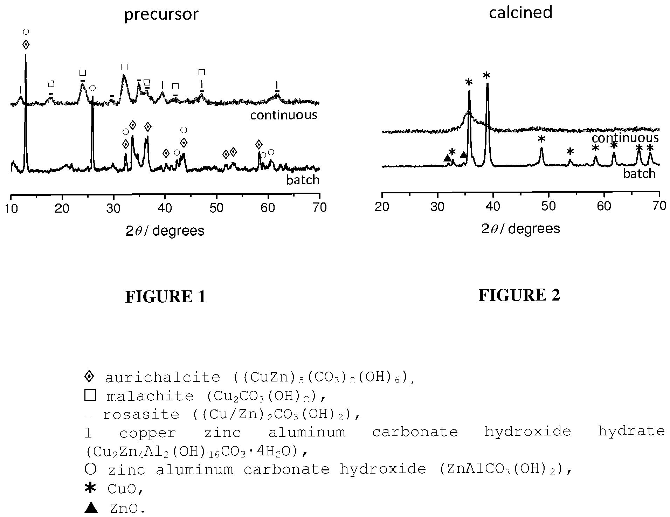

The resultant filter cakes were dried at 80°C for 12 h. X-ray diffraction (XRD) analysis confirmed the presence of malachite (Cu₂CO₃(OH)₂), rosasite (Cu/Zn)₂CO₃(OH)₂) and copper zinc aluminum carbonate hydroxide hydrate (ZnAlCO₃(OH)₂) as crystalline phases with aging, and amorphous phases without aging of the material (Figure 1). No formation of copper oxide (CuO), zinc oxide (ZnO), or mixtures thereof was observed in any case. The dried powder precursors were afterwards calcined in static air at 300°C for 3 h using a heating rate of 2°C min⁻¹. XRD analysis confirmed the presence of broad reflections of CuO, thus indicating a small particles size of CuO (Figure 2).

### Example 2 (comparative): Preparation of a Cu/ZnO/Al₂O₃ catalyst by batch co-precipitation

For comparison purposes, a catalyst precursor powder was prepared by batch co-precipitation with the molar ratio Cu:Zn:Al of 60:30:10, by precipitating at room temperature, pH = 8, and under mechanical stirring (500 rpm), a mixture of aqueous solutions of Cu(NO₃)₂·6H₂O, Zn(NO₃)₂·5H₂O, and Al(NO₃)₂·9H₂O using sodium carbonate (Na₂CO₃) as precipitating agent. Once the precipitation was completed (i.e. after about one hour), the slurry was aged at 60°C for 1 h under mechanical stirring (500 rpm). The slurry was then filtered and thoroughly washed with deionized water until the pH became neutral.

The resultant filter cake was dried at 80°C for 12 h. XRD analysis (see Figure 1) confirmed the presence of aurichalcite, (CuZn)₅(CO₃)₂(OH)₆, and zinc aluminum carbonate hydroxide, ZnAlCO₃(OH)₂.

The dried powder precursor was afterward calcined in static air at 300°C for 3 h using a heating rate of 2°C min⁻¹. XRD analysis indicated mainly the presence of highly crystalline CuO with higher particle size than the sample prepared in continuous mode (Figure 2). Low intensity reflections of ZnO can be also distinguished.

### Example 3: Properties of the catalysts

The calcined samples obtained in Examples 1 and 2 were reduced *in situ* for full activation by exposing them to a hydrogen-containing gas feed comprising 10% H₂ in nitrogen, initially at 150°C, with reduction performed at a maximum temperature of 175°C. In order to determine the copper particle size of the reduced catalyst, the reduction procedure was mimicked by temperature-programmed reduction with hydrogen (H₂-TPR). The analysis was carried out in a mixture of 5 vol.% H₂ in N₂, ramping the temperature from 160 to 230°C at 2°C min⁻¹.

For all the examples, the average copper oxide (*D*_{CuO}) and copper (*D*_{Cu}) particle size were estimated from XRD by application of the Scherrer method. The external surface area of the calcined materials (*S*_{BET}), as well as the copper surface area (*S*_{Cu}) of the reduced counterparts were determined by N₂ adsorption and N₂O chemisorption (or reactive frontal chromatography), respectively, according to the methods described by EVANS J.W. et al. in Appl. Catal. 1983, 7, 75-83.

The results are listed in Table 1 below.

**Table 1**

| **Sample** | **Aging time (h)** | ***S*_{BET} (m² g⁻¹)** | ***D*_{Cu} (nm)** | ***S*_{Cu} (m² g⁻¹)** |
|---|---|---|---|---|
| Continuous (Example 1) | 0 | 60 | 5 | 22 |
| | 1 | 60 | 5 | 20 |
| Batch (Example 2) | 1 | 22 | 19 | 4 |

These results clearly show that the particle size and surface area of the catalysts derived from the continuous mode, which are the subject of the present patent application, are superior to those derived from the batch-synthesized precursor. Both catalysts prepared with and without aging exhibited identical properties.

### Example 4: Catalytic test

### a) First set of conditions

A sample of each of the catalysts (0.4 g) from the continuous mode and batch mode, as obtained in Examples 1 and 2 and sieved in the range 0.125-0.3 mm, was loaded to a reactor as powder and reduced *in situ* according to the procedure described in Example 3. A methanol synthesis gas with a molar ratio composition of H₂/CO = 7 was passed over the catalyst at a pressure of 50 bars and at a temperature of 220°C. The outlet methanol was measured online using a gas chromatography system. The methanol formation rates of the catalysts of Example 1 were very similar i.e. 0.009 g_{MeOH} g(c)⁻¹h⁻¹ and 0.008 g_{MeOH} g(c)⁻¹h⁻¹, while the catalyst of Example 2 displayed a rate of 0.003 g_{MeOH} g(c)⁻¹h⁻¹. If we consider the batch-derived material a reference catalyst for methanol synthesis, then the catalytic performance of the catalyst obtained according to this invention can be expressed as relative to the benchmark catalyst. Accordingly, the results are as expressed in Table 2 below.

**Table 2**

| **Catalyst** | **Aging time (h)** | **Reaction time (h)** | **Relative activity** |
|---|---|---|---|
| Continuous (Example 1) | 0 | 20 | 3.0 |
| | 1 | 20 | 2.6 |
| Batch (Example 2) | 1 | 20 | 1.0 |

These results show superior activity of the catalyst derived from the precursor prepared by the continuous synthesis method of this invention, compared to the standard catalyst with the same Cu:Zn:Al molar ratio and tested under the same conditions. This higher activity is a consequence of the higher surface area and smaller particle size with respect to the typical catalyst.

### b) Second set of conditions

The same catalysts with the same properties were used for a new methanol synthesis reaction, but with different operating conditions than above. A methanol synthesis gas with a molar ratio composition of H₂/CO = 7 and CO₂ amount of 1.75% v/v present in the feed was passed over each of the catalysts at a pressure of 50 bars and at a temperature of 180°C. The methanol formation rates of the catalysts of Example 1 were identical, namely 0.007 g_{MeOH} g(c)⁻¹h⁻¹, while the catalyst of Example 2 displayed a rate of 0.002 g_{MeOH} g(c)⁻¹h⁻¹. As above, the catalytic performance of the continuous-derived catalyst of Example 1 can be expressed relative to that of the batch-derived catalyst of Example 2, as shown in Table 3.

**Table 3**

| **Catalyst** | **Aging time (h)** | **Reaction time (h)** | **Relative activity** |
|---|---|---|---|
| Continuous (Example 1) | 0 | 20 | 3.5 |
| | 1 | 20 | 3.5 |
| Batch (Example 2) | 1 | 20 | 1.0 |

As expected and in line with the results given in Table 2, the sample prepared by the continuous mode is a better catalyst for methanol synthesis than the reference material. Additionally, the methanol production relative activity increased compared to the data displayed in Table 2, indicating that the ternary system obtained according to this invention is a suitable catalyst for methanol synthesis in the presence of CO₂.

## Claims

1. A continuous process for the manufacture of a CuO/ZnO/Al₂O₃ catalyst, comprising the successive steps of:
(a) continuously co-reacting aqueous solutions of copper, aluminum and zinc salts with at least one base in a reaction vessel, under stirring, at a temperature ranging from 20 to 40°C, so as to form a slurry, wherein the reaction time is controlled and adjusted between 5 and 60 seconds and the pH of the slurry is adjusted between 7.5 and 8.5,
(b) optionally, subjecting said slurry to aging in a separate vessel,
(c) filtering and washing said slurry with water until a filter cake having a pH of 7 is obtained,
in such a way as to obtain crystalline metal oxide precursors at the end of step (b), if present, or at the end of step (c),
(d) drying said filter cake so as to obtain a precursor powder, and
(e) calcining said precursor powder at a temperature of less than 320°C to obtain the catalyst.

2. The process according to Claim 1, **characterized in that** the salts are chosen from: nitrates, carbonates, acetates and oxalates, preferably nitrates.

3. The process according to any of claims 1 and 2, **characterized in that** the base is selected from carbonates such as sodium carbonate, ammonium carbonate and potassium carbonate; mixtures of at least one hydroxide such as sodium hydroxide and ammonium hydroxide with at least one carbonate; and mixtures of at least one hydroxide such as sodium hydroxide and ammonium hydroxide with at least one bicarbonate, wherein sodium carbonate is preferred.

4. The method according to any of claims 1 to 3, **characterized in that** stirring is performed at a speed of from 10.000 to 25.000 rpm.

5. The method according to any of claims 1 to 4, **characterized in that** the reaction time is adjusted between 10 and 30 seconds, preferably between 15 and 20 seconds.

6. The method according to any of claims 1 to 5, **characterized in that** the aging step, if present, and the washing step are performed during a combined period of time of at least 15 minutes.

7. The method according to any of claims 1 to 6, **characterized in that**, in the crystalline metal oxide precursors, copper is only included in malachite-type compounds of formula CuMCO₃(OH)₂ where M is Cu or Zn.

8. The method according to any of claims 1 to 7, **characterized in that** the crystalline metal oxide precursors are malachite, rosasite and copper zinc aluminum carbonate hydroxide hydrate.

9. The method according to any of claims 1 to 8, **characterized in that** the metals are mixed in respective molar amounts of from 30 to 75 mol.% of Cu, from 20 to 60 mol.% of Zn and 5 to 20 mol.% of Al, provided that the total molar amount of Cu, Zn and Al is 100%, preferably in respective molar amounts of Cu/Zn/Al of 60:30:10.

10. A catalyst obtainable according to the process as claimed in any of claims 1 to 9.

11. The catalyst of claim 10, **characterized in that** it has an external surface area of at least 40 m²/g, preferably of at least 50 m²/g.

12. The catalyst of any of claims 10 and 11, **characterized in that** it has, after reduction, a copper surface area of more than 16 m²/g and preferably of more than 20 m²/g.

13. The catalyst of any of claims 10 to 12, **characterized in that** the copper oxide crystallites have an average particle size of less than 15 nm, preferably less than 10 nm and more preferably less than 7 nm.

14. Use of a catalyst according to any of claims 10 to 13 in the synthesis of methanol from syngas or in a water gas shift reaction.
